# EUROPEAN PATENT APPLICATION

(11) **EP 1 941 946 A1**
(43) Date of publication of application: **09.07.2008**
(21) Application number: 07000116.9
(22) Date of filing: 04.01.2007
(51) Int. Cl.: B01J 27/00, B01J 27/20, B01J 27/24, C07C 2/48, C07C 68/00, C07D 251/00, C07C 37/60

(54) **Carbonitrides as catalysts**

(71) Applicant: Max-Planck-Gesellschaft zur Förderung der Wissenschaften e.V., 80539 München (DE)
(72) Inventor: Antonietti, Markus, 14558 Nuthetal OT Rehbrücke (DE); Goettmann, Frederic, 14545 Werder / Havel (DE); Thomas, Arne, 10829 Berlin (DE); Fischer, Anna, 56290 Beltheim (DE)
(74) Representative: HOFFMANN EITLE

(57) **Abstract**

The present invention relates to the use of a preferably transition metal-free carbonitride as a catalyst, and to a supported catalyst comprising the said carbonitride. The carbonitride catalysts proved effective in the cyclization of organic compounds, in particular in the cyclodimerization and cyclotrimerization of alkynes and nitriles to yield aromatic products. Moreover, the catalyst was shown to be effective in the direct conversion of aromatic compounds with carbon dioxide or a carbonyl compound to the corresponding phenolic compound. Finally, the invention is concerned with the use of the catalyst for the preparation of certain disubstituted organic carbonates. Since the catalysts of the invention do not require the presence of e.g. transition or noble metals, they are environment-friendly and inexpensive while allowing the above types of reactions to be carried out at high conversion rates, and with high regioselectivity.

## Description

### FIELD OF THE INVENTION

The present invention is concerned with carbonitrides, preferably metal-free carbonitrides as catalysts, such as for organic cyclization reactions, the direct conversion of aromatic compounds with carbon dioxide or a carbonyl compound to afford the corresponding phenolic compound, and the synthesis of disubstituted organic carbonates by reacting carbon monoxide with suitable alcohols.

### BACKGROUND OF THE INVENTION

Most industrial processes involve the use of catalysts, in particular heterogeneous catalysts. For reasons of stability and reactivity, most catalysts are inorganic catalysts.

WO 2005/016519, US 2005/0176989 and US 2005/0176990, all to Mosanto Technology LLC, are concerned with processes for the oxidation of organic substrates with an oxidizing agent in the presence of an oxidation catalyst. The oxidation catalyst comprises a carbon support having formed thereon a transition metal composition comprising a transition metal, nitrogen, and carbon. In certain embodiments, the catalyst may include a transition metal carbide-nitride, e.g. cobalt or iron carbide-nitride. Typically, the transition metal constitutes at least about 0.1 wt.-% of the oxidation catalyst, more typically ≥ 0.5 wt.-%, still more typically ≥ 1 wt.-%. These catalysts are stated as being useful in the oxidation of tertiary amines to secondary amines, and for the oxidation of formaldehyde to formic acid. In the catalysts described in the above patent applications, the catalytic effect critically depends on the presence of the transition metal.

Cyclization reactions, in particular those yielding aromatic cyclic compounds play an important role in organic industrial synthesis. For instance, the trimerization of acetylenes is a valuable synthetic tool because it enables the preparation of functionalized aromatic rings. See S. Saito, Y. Yamamoto, Chemical Reviews 2000, 100, pages 2901-2915. Many proposals of catalysts based on transition metals have been made for catalyzing this type of reaction. See S. Kotha et al., European Journal of Organic Chemistry 2005, pages 4741-4767. In industry, processes relying on Ziegler-Natta-catalysts are particularly attractive because these compounds are well-characterized and readily available. This is reported by E.F. Lutz in the Journal of the American Chemical Society 1961, 83, pages 2551-2554.

The synthesis of triazines by trimerization of nitriles is a related cyclization process. It is equally important since these compounds represent important intermediates in polymer chemistry. See A. Diaz-Ortiz et al., Green Chemistry 2002, 4, pages 339-343 and A.M. Gupta, C.W. Macosko, Macromolecules 1993, 26, pages 2455-2463. Most syntheses use strong bases such as sodium hydride (X.C. Tao et al., Journal of Molecular Catalysis A-Chemical 2003, 201, pages 155-160) or molten sodium hydroxide (T. Murase, M. Fujita, Journal of Organic Chemistry 2005, 70, pages 9269-9278). Triazine derivatives are used as monomers and cross-linkers for thermosetting materials and lacquer coatings, and are presently being explored as sunscreen agents.

As will be appreciated from the above, the trimerizations of acetylen and nitriles of the prior art involve the use of expensive transition metals, which may further be of doubtful toxicity, or highly aggressive reactants, such as strong bases. This is problematic in view of the protection of the environment and does not fulfil the requirements of Green Chemistry. Furthermore, the regioselectivity is difficult to control in these syntheses and many functional groups are prone to decomposition under the severe reaction conditions used. Consequently, alternative catalysts allowing educts having various functional groups to be converted with high regioselectivity were in high demand.

Dimethyl carbonate (DMC) is an environmentally benign chemical product with a wide range of applications. DMC can serve as a non-toxic substitute for dimethyl sulfate and phosgene, which are highly toxic and corrosive methylation and carbonylating agents. In addition, DMC is also a good solvent, a useful fuel additive and a monomer for the synthesis of polycarbonate resins. One synthesis method of DMC requires phosgene as a reagent. This is highly undesirable due to the extreme toxicity of phosgene. Another route to the preparation of DMC is the oxidative carbonylation of CH₃OH with CO and oxygen. However, both copper and palladium catalysts are required for this synthesis. The use of expensive metal catalysts makes the process unfavourable especially on an industrial scale. In recent years, direct synthesis of DMC from CO₂ and CH₃OH became an attractive approach. See X.L. Wu et al., J. Mol. Catal. A-Chem. 2006, 249, pages 93-97.

However, the direct synthesis of disubstituted organic carbonate derivatives from carbon monoxide and the corresponding alcohol without the need of any oxidant and any metal catalyst such as copper or palladium was not yet feasible.

Several transition metal catalyzed approaches to activate CO₂ have been explored (see D. Walther et al., Coord. Chem. Rev. 1999, 182, 67-100, and X.L. Yin, J.R. Moss, Coord. Chem. Rev. 1999, 181, 27-59). Previous metal-free attempts to synthetically sequester CO₂ concentrated on the use of alkylated amidines and guanidines, such as 1.8-diacabicyclo[5.4.0]undec-7-ene(DBU)(see M. Costa et al., J. Chem. Soc.-Perkin Trans.1 1998, 1541-1546, E. Haruki et al., Chem. Lett. 1974, 427-428, W.D. McGhee et al., Organometallics 1993, 12, 1429-1433 and E.R. Perez et al., Tetrahedron Lett. 2002, 43, 4091-4093). However, the direct synthesis of phenolic compounds, such as phenol, by reacting the corresponding aromatic compound, such as benzene, with carbon dioxide in the presence of a heterogeneous metal-free catalyst was considered impossible.

In view of the above, it is an object of the present invention to provide a new catalyst which is friendly to the environment and inexpensive in that it does not contain substantial amounts of metals such as transition metals or noble metals. It is another object to provide a catalyst enabling the cyclization of organic compounds, in particular the trimerization of alkynes and nitriles which allows the environment-friendly synthesis of cyclic compounds, in particular aromatic compounds, carrying substituents sensitive to harsh reaction conditions, which compounds were not readily accessible via the synthetic approaches of the prior art. A still further object of the invention resides in the provision of a catalyst allowing the synthesis of disubstituted organic carbonates, such as DMC using carbon monoxide as a starting compound without need of any oxidant. A still further object resides in the provision of a catalyst for direct conversion of an aromatic compound, such as benzene, with carbon dioxide to afford the corresponding phenolic compound, such as phenol. As regards the above catalysts useful for certain processes, the present invention also aims at the processes using such catalysts, in particular environment-friendly and inexpensive catalysts, as such.

### SUMMARY OF THE INVENTION

The above objects can all be achieved by the use of a carbonitride, preferably a metal-free carbonitride as a catalyst. For instance, it has now been surprisingly found that highly condensed graphitic carbonitrides having a structure close to graphitic C₃N₄ are highly effective catalysts for various types of reactions, including those outlined below.

A first process which can be catalyzed with the catalyst of the present invention is the cyclization of at least one organic compound each having at least one triple bond. In another aspect, the catalyst is used for converting an aromatic compound with carbon dioxide or a carbonyl compound to provide the phenolic compound corresponding to the aromatic compound. A third aspect is the preparation, using the said catalyst, of a disubstituted organic carbonate derivative having the following formula (IV) 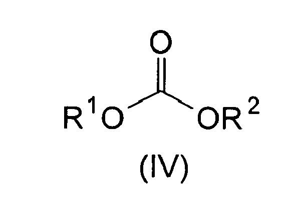 wherein the substituent meanings are as defined in the appended claims.

Preferred embodiments of the catalysts and processes using the same in accordance with the present invention are subject of the dependent claims.

### BRIEF DESCRIPTION OF THE DRAWINGS

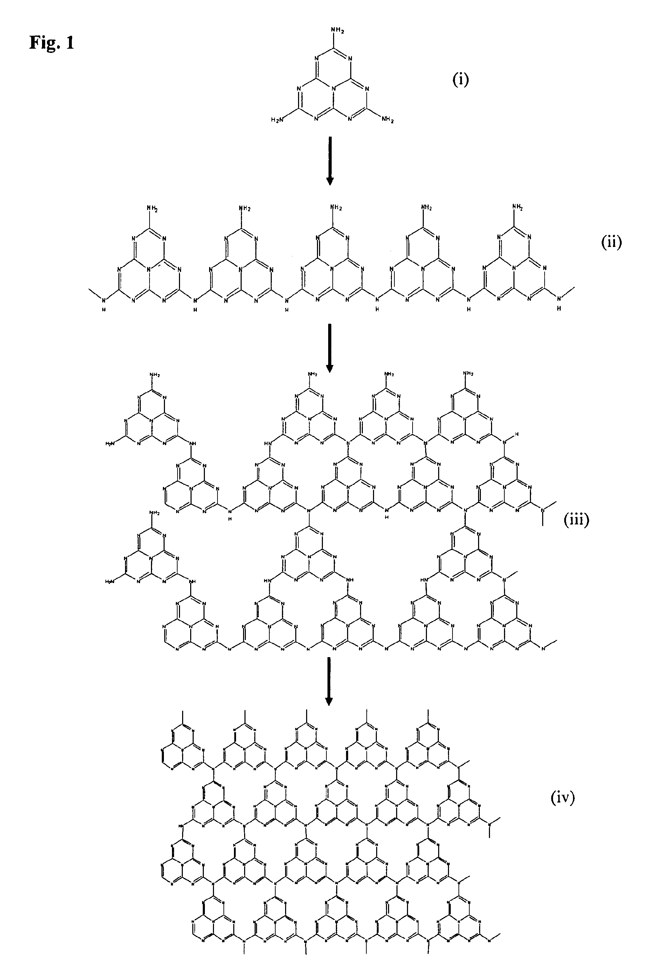

Fig. 1 provides a schematic graph showing the condensation of melem (formula (i)) represented by the formula C₆N₁₀H₆ via "melon" (formula (ii)) represented by formula C₆N₉H₃ and the highly condensed graphitic carbonitride (formula (iii)) represented by the formula C₆N₈₊ₓH_{y} (wherein an x is 0 to 1 and y is 0 to 3), to the final "ideal" graphitic carbonitride (formula (iv)) represented by formula C₃N₄.

### DETAILED DESCRIPTION OF THE INVENTION

The term "carbonitride" as it is used herein, means a solid comprising carbon and nitrogen atoms without substantial amounts of further metals such as transition metals incorporated in the network of the solid. As such, a carbonitride is to be distinguished from a transition metal carbide-nitride as *e.g.* described in WO 2005/016519, US 2005/0176989 or US 2005/0176990, in which the transition metal constitutes at least 0.1 % by weight of the oxidation catalyst. Unlike the metal carbide-nitrides of the above patent applications, the carbonitride for use as a catalyst in the present invention contains less than 0.1 %, preferably less than 0.05 %, more preferably less than 0.01 % and still more preferably less than 0.001 % by weight of metal, such as transition metal. However, the "carbonitrides" as this term is used herein, may contain hydrogen, for instance up to 6 %, preferably below 3 % by weight of hydrogen.

According to a preferred embodiment, the carbonitride is "metal-free" in the sense that no metal e.g. transition metal or noble metal was intentionally added during or after the synthesis thereof to be incorporated into the network of the solid. Consequently, "metal-free" also covers those situations in which unavoidable metal impurities are contained in the carbonitride structure.

The metal-free carbonitride is not specifically limited in kind as long as it is active as a catalyst in the wanted reaction. For instance, it may be a solid obtained by condensation of melem units. Melem represented by the formula C₆N₁₀H₆ is shown as formula (i) in Fig. 1. During the condensation of monomeric melem units to afford oligomeric and ultimately polymeric solids, ammonia is liberated. The condensation can be understood to proceed via a linear polymeric or oligomeric molecule referred to as "melon" which can be represented by formula C₆N₉H₃ (see formula (ii) in Fig. 1). Further condensation yields a highly but not yet completely condensed graphitic carbonitride, schematically illustrated by formula (iii) in Fig. 1. The theoretical endpoint of the condensation is graphitic carbonitride having the empirical formula C₃N₄ as shown by formula (iv) in Fig. 1. In the present invention, in particular for catalyzing the distinct reactions as identified above, a highly condensed graphitic carbonitride is used with preference. The "highly condensed graphitic carbonitride" (as the term is used herein) is represented by the formula C₆N₈₊ₓH_{y} with x being from 0 to 1, and y from 0 to 3. Such a compound is schematically illustrated by formula (iii) in Fig. 1. It is a compound forming a planar two-dimensional structure. The condensation degree is between "melon" and fully condensed ideal graphitic carbonitride C₃N₄. However, the ideal structure which can be represented by the formula C₃N₄ (corresponding to C₆N₈), i.e. x and y are both 0 in the above formula C₆N_{8+X}H_{Y'} can hardly be reached in real life synthesis. This being the case, the highly condensed graphitic carbonitride is usually represented by the formula C₆N₈₊ₓH_{y}, wherein x is in the range of from above 0 to 1 and y is in the range of from above 0 to 3. Attaining as high a condensation degree as possible is preferred in the present invention. So, x is preferably in the range of from above 0 to 0.2, preferably in the range of from above 0 to 0.1 and y is preferably in the range of from above 0 to 0.6, more preferably in the range of from above 0 to 0.3. As will be appreciated, the above preferred types of highly condensed graphitic carbonitrides have a structure close to ideal graphitic C₃N₄. For convenience, such material is occasionally named "graphitic C₃N₄" hereinafter even though it does not have the stoichiometry of C₃N₄ in the strict sense but contains low amounts of hydrogen due to unreacted NH₂ groups of the constituent melem units.

Other useful carbonitrides are those represented by the formula C₁NₐH_{b}, wherein a is 0.5 to 2 and b is 0 to 1. An example of such a carbonitride is C₃N₃. As is the case for the above graphitic carbonitrides, the formula C₃N₃ allows for the presence of minor amounts of hydrogen.

As the carbonitrides of the invention, e.g. the highly condensed graphitic carbonitrides, possess a high degree of inertness and are insoluble in all known solvents, in particular in the solvents normally used for catalytic reactions, they are useful as heterogeneous catalysts.

Preferably, in the order of preference, at least 90 %, at least 95 %, at least 98 %, at least 99 %, or at least 99.9 % of the catalyst of the invention consist of carbonitride. According to one embodiment of the invention, pure carbonitride (which allows for the presence of inevitable impurities originating from the preparation thereof) is used as the catalyst.

Preparation methods of highly condensed graphitic carbonitrides, such as graphitic C₃N₄, which are useful as catalysts in the present invention will be explained below.

Generally, highly condensed graphitic carbonitrides can be prepared from suitable molecular precursor compounds, e.g. by self-condensation of cyanamide, dicyandiamide, ammonium dicyanamide or melamine accompanied by elimination of ammonia. For instance, graphitic C₃N₄ can be synthesized by tempering one of the above starting compounds or a mixture thereof, preferably dicyandiamide, in a vessel which is covered but allows the formed ammonia to evolve. The heat treatment conditions are not specifically limited and a suitable temperature is in the range of 400 to 700 °C, preferably from 500 to 600 °C. There is no specific restriction as to the time of heat treatment. It may be for example 1 to 10 h. The pressure conditions are also not specifically limited, and the pressure may be from 10⁻² to 10 bars, and preferably the pressure is atmospheric pressure. While the cover of the vessel is loose enough to allow the evolution of ammonia, it is preferably tight enough to prevent the sublimation of the starting compound, e.g. melamine, or the oxidation of the carbonitride by atmospheric oxygen. To give an example of a preparation method, heating 10 g dicyandiamide in a covered ceramic crucible from a temperature to 550 °C in two hours and keeping this temperature for 4 h will afford graphitic C₃N₄. To reach higher condensation degrees of highly condensed graphitic carbonitrides, it can be advantageous to work in a sealed vessel under vacuum (e.g. at about 10⁻² bars) at a high temperature such as in the range of from 600 to 700 °C.

More details of the synthesis of highly condensed graphitic carbonitrides can be taken from B. Jürgens et al., J. Am. Chem. Soc. 2003, 125, pages 10288-10300, which scientific article is incorporated by reference herein. As will be appreciated by one of average skill in the art, the above thermal treatment of molecular precursor compounds can be effected in any vessel, not only in ceramic crucibles, but also in glass or quartz ampoules as long as these vessels can be covered or sealed and the vessel material is sufficiently stable and inert under the reaction conditions used.

As large surface areas are advantageous in heterogeneous catalysis, the carbonitrides of the present invention preferably have a porous structure, in particular a mesoporous or macroporous structure. It is preferred to use as the catalyst of the invention a mesoporous or macroporous highly condensed graphitic carbonitride, such as a graphitic C₃N₄, and it is more preferred to use a mesoporous graphitic C₃N₄.

Porous graphitic C₃N₄ can be prepared by modifying the synthesis as described above. For instance, it can be obtained by the self-condensation of the molecular precursor compounds as identified above in the presence of hard templates. Useful hard templates are particles of the appropriate size, i.e. 2 to 50 nm for mesoporous materials and > 50 nm for macroporous materials, which are capable of surviving the tempering treatment for self-condensation of the precursor compounds to afford the graphitic C₃N₄ structure, and which can be removed after condensation while leaving intact the porous network. Useful are for instance silica nanoparticles (e.g. Ludox^{®}, Aldrich; Nalco^{®} manufactured by Nalco Chem USA).

Preferably, the silica nanoparticles used as templates can be dispersed homogeneously in the mixture, for instance the melt, of the molecular precursor compounds, e.g. in cyanamide, dicyandiamide, ammonium dicyanamide or melamine. The dispersion of the particles may be favoured by appropriate surface interactions between the silica surface and the amine and, later, when the condensation proceeds, aromatic nitrogen groups. Thereby, the mass ratio between the silica particles and the molecular precursor compound can be varied in broad ranges. For instance, the mass ratio between the molecular precursor compound, such as cyanamide and silica nanoparticles can vary from 0.5 to 1.6.

After completion of the self-condensation of the precursor compounds, the hard templates are removed to afford the porous material. This can be accomplished by using any agent capable of dissolving the hard template incorporated in the highly condensed graphitic carbonite structure provided the structure is inert to the agent. In the case of using silica nanoparticles as hard templates, suitable agents are e.g. aqueous HF or aqueous NH₄HF₂ with the latter being preferred. The concentration of NH₄HF₂ in the aqueous NH₄HF₂ may be for example, without limitation, from 1 M to 8 M, and it is preferably about 4 M. Furthermore, using strong basic solutions such as ammonia, NaOH or KOH solutions, to dissolve the silica particles is an option.

The powders of porous graphitic C₃N₄ can then be recovered from the agent for removing the hard template, e.g. by decantation or centrifugation. Subsequently, the powders may be further treated, e.g. washed, and dried and comminuted. There are no specific limitations as to the washing, in terms of washing agents, washing time and numbers of washings. Suitable washing agents are for instance water and ethanol. The drying may be carried out under vacuum.

An alternative approach for increasing the surface area of the carbonitrides, specifically of the highly condensed carbonitrides is the use of porous silicates as templates for the generation of graphitic C₃N₄ nanoparticles. Owing to their small size, these nanoparticles have a large specific surface area. The porous silicates used as templates are preferably mesoporous silicates, such as for example MCM-41, MCM-48, SBA-15, and MCF- and MSU-type mesoporous silicates (or silicas).

By way of the above method using hard templates such as silica particles, highly condensed graphitic carbonitride, in particular graphitic C₃N₄ having a specific BET surface area of 50 to 500 m²/g, preferably 100 to 400 m²/g, most preferably 150 to 350 m²/g can be obtained.

As it turned out, the catalytic performance of the highly condensed graphitic carbonitrides was hardly affected by variations in the synthesis thereof.

The carbonitride for use as a catalyst in the present invention may be used in unsupported form or supported form. According to one aspect, the present invention is concerned with supported carbonitride catalysts as such, which comprise a supporting material, and carbonitride provided thereon. It is to be understood that the carbonitride provided on the supporting material is a carbonitride as detailed above. Suitable supporting materials are those commonly used in catalysis, e.g. materials having a high specific surface area, such as active carbon, alumina, silica, silicates, etc. According to one embodiment of the supported carbonitride catalyst of the invention, silica powder such as silica microspheres, in particular silica mesopouros microspheres are used as a supporting material.

For reasons of catalytic activity, the specific surface area of the supported carbonitride catalyst should be as high as possible. For instance, the specific BET surface area (determined by nitrogen adsorption) is in the range of 100 to 350 m²/g, preferably it is 150 to 300 m²/g and most preferably it is 200 to 300 m²/g.

The supported carbonitride catalyst of the invention may comprise, as a carbonitride, a highly condensed graphitic carbonitride, preferably graphitic C₃N₄, *e.g.* in mesoporous form.

There are no specific limitations of the method of preparing a supported carbonitride catalyst according to the invention. A suitable method may comprise the following steps:
(i) dispersing the supporting material in a solution of the molecular precursor compounds of the carbonitride to infiltrate the molecular precursor compounds in the supporting material;
(ii) recovering the infiltrated supporting material from the solution; and
(iii) heat treating the recovered infiltrated supporting material to convert the molecular precursor compounds to the carbonitride.

The dispersing step (i) to allow the molecular precursor compounds of the carbonitride to permeate into, i.e. to infiltrate the supporting material may be assisted by suitable means such as agitation (e.g. stirring) or sonication. To facilitate the dispersion, the supporting material preferably is in communuted form such as in the form of powder. As will be appreciated, the amount of molecular precursor compound of the carbonitride (e.g. of cyanamide, dicyandiamide, ammonium dicyanamide or melamine) infiltrated in the supporting material depends on the absorption capability of the supporting material for the solution of the molecular precursor compounds, as well as the concentration of the solution.

The solvent in the solution of the carbonitride molecular precursor compounds may be any solvent as long as this is capable of dissolving the selected precursor compound. For instance, the solution may be an aqueous solution. The term "aqueous" as used herein means a solution which contains water as a main solvent with the optional addition of further solvents that are miscible with water. The infiltrated supporting material may be recovered from the solution by usual techniques for separating solid-liquid-mixtures, *e.g.* by filtration or centrifugation. Suitable heat treatment conditions for converting the precursor compounds to the carbonitride. e.g. graphitic C₃N₄ may be as explained previously for the unsupported carbonitrides.

The present inventors found out that the specific surface area of the obtained supported carbonitride catalyst can be influenced by the concentration of the molecular precursor compound of the carbonitride in the solution of step (i). Specifically, the mass ratio of the molecular precursor compound to water in an aqueous solution of the said precursor was shown to be of influence, here. Lower amounts of the precursor compound, such as cyanamide, in relation to water in the aqueous solution increased the BET specific surface area of the resulting supported carbonitride catalyst. Consequently, the mass ratio of the molecular precursor compound, such as cyanamide, while it is not specifically limited and may for instance be in the range of (mass ratio) 5:1 to 1:5 (molecular precursor compound: water), is preferably in the range of 1:1 to 1:4 and still more preferably 1:2 to 1:4.

According to one aspect, the carbonitride catalyst of the present invention is used in a process for the cyclization of at least one organic compound each having at least one triple bond. In case one organic compound having at least two triple bonds, preferably two or three triple bonds is subjected to the catalytic process, the cyclization may occur within the molecule, e.g. intramolecular. In the alternative, the cyclization may occur between two or three, most preferably three organic molecules, each having one triple bond. The latter intermolecular cyclizations (also referred to as cycloadditons) are preferred in the present invention. Most preferably, the two or three, preferably three organic compounds undergoing the cyclization are the same compounds.

According to another specific embodiment, the triple bond, preferably the one triple bond of the two or three, preferably three organic molecules undergoing the cyclization is a carbon-carbon or carbon-nitrogen triple bond. As usual, compounds having (at least one) carbon-carbon triple bond are also referred to as alkynes, and compounds having (at least one) carbon-nitrogen triple bond as nitriles. Also, mixtures of alkynes and nitriles may be subjected to the cyclization (such as cyclodimerization and cyclotrimerization reaction).

Cyclization reactions such as cyclotrimerization reactions are quite distinct from other types of reactions such as Friedel Crafts reactions, e.g. in terms of the catalyst used. While Lewis acids such as AlCl₃, lanthanides or zeolites are usually used as catalysts in Friedel Crafts reactions, cyclotrimerizations have usually been catalysed with transition metals as yet.

The alkyne to be subjected to the cyclization may be selected from the group consisting of monophenyl acetylene, diphenyl acetylene, dimethylacetylene dicarboxylate, propargyl alcohol, or a mixture thereof. The nitrile may be selected from the group consisting of alkyl carbonitriles, aryl carbonitriles, and heteroaryl carbonitriles, or a mixture thereof. Specifically, the nitrile may be selected from the group consisting of acetonitrile, propionitrile, 3-chloro-propionitrile and pyrazine carbonitrile, or a mixture thereof.

The product of the cyclization process preferably is an aromatic compound. Most preferably, the product of the cyclization is represented by one of the following formulae (I) to (III):

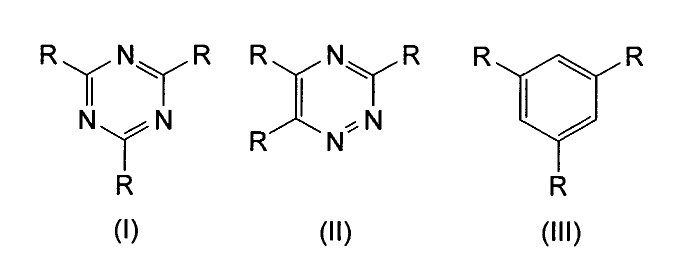

In the formulae, the substiutents R can be independently selected from optionally substituted alkyl, aryl, heteroaryl, and ester groups CO₂A with A representing alkyl or aryl.

The above optionally substituted alkyl group and the alkyl group A may be a linear or branched C₁₋₆ alkyl group. It is preferably a C₁₋₃ alkyl group and most preferably methyl or ethyl.

The above optionally substituted aryl group and the aryl group A may be a C₃₋₁₀ aryl group. Preferably, it is a C₅₋₆ aryl group, more preferably a phenyl group.

The optionally substituted heteroaryl group R in the above formulae may be a C₃₋₁₀ heteroaryl group. It is preferably a C₅₋₆ heteroaryl group. The heteroaryl group preferably has one or two heteroatom(s) independently selected from nitrogen (N), sulphur (S), oxygen (O) and phosphorus (P). The heteroaryl substituent may for instance be a pyrazine substituent, such as a 2-pyrazinyl group.

The optional substituents are not specifically limited in kind and may for instance be halo (such as chloro, bromo), hydroxyl, carboxy, alkyl (such as specified above), aryl (such as specified above) and heteroaryl (such as specified above).

Most preferably, the three substituents R attached to the aromatic ring as shown e.g. in the formulae (I), (II) and (III) are each the same. As will be appreciated, this is a consequence of using the same organic compounds, i.e. a mixture comprising a single type of organic molecules having a triple bond as starting compounds in the cyclization process.

As can be seen, the organic staring compounds yielding upon cyclization aromatic compounds as represented by the above formulae (I) to (III), may be mono and dialkyl acetylenes, mono and diaryl acetylenes, mono and diheteroaryl acetylenes, and acetylene diesters, with the alkyl, aryl, heteroaryl groups and optional substituents as specified above. Preferably, the starting compounds are di(C₁₋₃)acetylenes.

The following cyclization reactions affording aromatic products are particularly preferred:
1. cyclization of acetonitrile (cyclotrimerization) to 2,4,6-trimethyl-1,3,5-triazine;
2. cyclization of acetonitrile (cyclotrimerization) to 3,4,6-trimethyl-1,2,5-triazine;
3. cyclization (cyclotrimerization) of propionitrile to 2,4,6-triethyl-1,3,5-triazine;
4. cyclization (cyclotrimerization) of pyrazine carbonitrile to 2,4,6-tripyrazino-1,3,5-triazine;
5. cyclization (cyclotrimerization) of 3-chloro-propionitrile to 2,4,6-(2-chloroethyl)-1,3,5-triazine;
6. cyclization (cyclodimerization and cyclotrimerization) of phenyl-acetylene to yield a mixture of 1-phenyl-naphthalene (dimer) and 1,3,5-triphenyl-benzene (trimer);
7. cyclization (cyclodimerization) of diphenyl-acetylene to give 1,2,3-Triphenyl-naphthalene;
8. cyclization (cyclotrimerization) of dimethyl-acetylene-dicarboxylate to benzenehexacarboxylic methylester;
9. cyclization (cyclotrimerization) of propargyl alcohol to 1,3,5-trihydroxymethyl benzene.

The above-listed cyclization reactions are preferably carried out using mesoporous highly condensed graphitic carbonitride, particularly mesoporous graphitic C₃N₄, as a catalyst.

According to another aspect of the present invention, the carbonitride catalyst is used for the preparation of a phenolic compound, which comprises the reaction of the aromatic compound to the corresponding phenolic compound with carbon dioxide or a carbonyl compound in the presence of the carbonitride catalyst.

As used herein, "phenolic compound" is intended to mean any aromatic compound substituted directly on the aromatic nucleus with at least one hydroxyl group (OH). Examples of the aromatic compound to be reacted in the above process are benzene, toluene, xylene, mesitylene, ethylbenzene and cumene, with benzene being preferred. The aromatic compounds may carry substituents, as long as these do not prevent a good adsorption of the aromatic compounds to the carbonitride catalyst, e.g. on the C₆N₈-units of the highly condensed graphitic carbonitrides of the invention. Suitable substituents are, without restriction, methyl, ethyl and n- and i-propyl. An exemplary reaction of a substituted aromatic compound may be the reaction of toluene to hydroxytoluene. However, for steric reasons, it is preferred to use unsubstituted aromatic compounds. Exemplary reactions are the reaction of benzene to phenol, and the reaction of benzene to dihydroxybenzenes, such as catechol.

The process may be carried out in the liquid phase, and in this case suitable solvents are commonly used solvents which are capable of dissolving both the aromatic compound to be reacted and the source of CO₂ or the carbonyl compound. When the aromatic compound is a liquid, and capable of dissolving the carbon dioxide source or the carbonyl compound, the reaction may be carried out in liquid phase without any additional solvent.

In the case of carbon dioxide as a reactant, useful sources of CO₂ are without restriction gaseous CO₂ and hydrogen carbonates which will decompose under the reaction conditions to generate carbon dioxide. Suitable hydrogen carbonates are for instance NaHCO₃ and KHCO₃.

Moreover, the presence of a base, in particular a strong base having a pKₐ of > 10, preferably ≥ 11 proved beneficial in the process involving the reaction of the aromatic compound with CO₂. Examples of useful bases, e.g. for the conversion of benzene to afford phenol, are hydrogen carbonates, such as NaHCO₃ and KHCO₃, alkali bases (e.g. NaOH, KOH) nitrogen based bases *(e.g.* NH₃, trimethyl, triethyl and tripropyl amines), and alcoholates (e.g. MeONa, MeOK, EtONa, EtOK). As will be appreciated, hydrogen carbonates, such as NaHCO₃ and KHCO₃ are useful bases while at the same time acting as a source of CO₂. So no further base needs to be added to the reaction mixture when hydrogen carbonates are used as carbon dioxide sources. This is why they are used with preference.

As used herein, "carbonyl compound" means a preferably organic compound containing a carbonyl (C=O) group. As such, it is a collective term including carboxylic acids, aldehydes, ketones and amides.

The present invention covers the four aspects that an aromatic compound is reacted with either one of a carboxylic acid, an aldehyde, a ketone, an amide, or a mixture thereof, to give, in the presence of the carbonitride catalyst of the invention, the corresponding phenolic compound.

As significant sterical hindrance at the carbonyl centre may reduce the reactivity of the carbonyl compound with the aromatic compound, the total number of carbon atoms in the carbonyl compound (inclusive of the carbonyl carbon atom) is preferably in the range of 1 to 10, more preferably 2 to 10 and especially 2 to 6 in the case of carboxylic acids, amides and aldehydes. The total carbon number of the ketones preferably is 3 to 10, more preferably 3 to 6, again including the carbonyl carbon atom. The above carbon atoms may form optionally substituted alkyl and/or optionally substituted aryl groups. According to a preferred embodiment, the carbonyl compound carries a proton, in particular a relatively labile proton, at the carbon in α-position of the carbonyl carbon atom.

In the case of the carboxylic acid, acetic acid is preferred. Amongst the aldehydes, acetaldehyde and hexanal may be exemplified, and acetaldehyde is used with preference.

According to a third aspect, the present invention is concerned with a method for the preparation of a disubstituted organic carbonate represented by formula (IV): 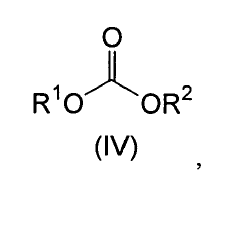 wherein R¹ and R² are the same or different and can be selected from the group consisting of alkyl, aryl and heteroaryl, and can mutually combine to form a ring structure containing the -OCOO-moiety, which comprises reacting carbon monoxide with at least one corresponding alcohol in the presence of the catalyst of the present invention. The above alkyl, aryl and heteroaryl groups can be substituted. Preferred alkyl, aryl, heteroaryl and substituent groups are the same as indicated for group R in the above formulae (I), (II), and (III).

When R¹ and R² combine to form a ring structure containing the -OCOO-moiety, the resulting compound is a cyclic organic carbonate. The ring structure is not specifically limited in size. However, it preferably is a 5- or 6-membered ring structure. The ring structure containing the -OCOO- moiety can further carry a substituent. Again, suitable substituent groups are as exemplified for group R in the above formulae (I), (II), and (III).

Consequently, cyclic organic carbonates represented by formula (IV) may be represented by the following formulae (V) and (VI).

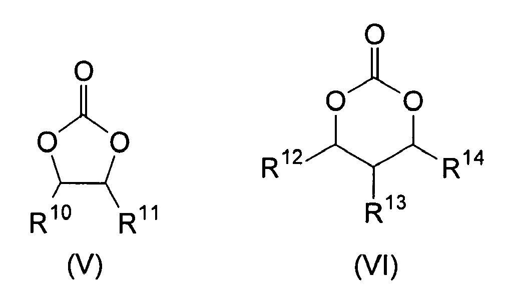

In the above formulae, R¹⁰, R¹¹, R¹², R¹³ and R¹⁴ are the same or different and can be selected independently from the substituents mentioned for group R in the above formulae (I), (II), and (III).

The most preferred reaction is the conversion of carbon monoxide and methanol to dimethyl carbonate. The above process offers the advantage that it can be carried out in the absence of any oxidant. The reaction conditions for converting carbon monoxide and methanol to dimethyl carbonate can be varied in broad ranges. For instance, when CO gas is used, the CO partial pressure may be 1 to 70 bar, preferably 1 to 20 bar, more preferably 1 to 10 bar. Alternatively, urea can be used as a source of carbon monoxide. The reaction temperature is preferably in the range of 100 to 200 °C, more preferably 130 to 170 °C, most preferably 140 to 160 °C. The reaction time may for instance be 10 to 72 h, preferably it is 10 to 50 h. The reaction can be carried out both in liquid and vapour phase. In case it is carried out in the liquid phase, the reaction solvent is not specifically limited in kind as long as it is capable of dissolving the reactants. For instance, the solvent may be an alcohol. Preferably, the alcohol is the alcohol to be reacted with carbon monoxide, i.e. no extra solvent is added.

The above disubstituted organic carbonates of formula (IV) can be further reacted, using the carbonitride catalysts of the invention, to provide the corresponding ethers. For instance, dimethyl carbonate can react with benzene to yield anisole and methyl formiate. The above consecutive reaction is similar to the reaction of aromatic compounds with carbon dioxide to provide the corresponding phenolic compound as described above.

In the following, the present invention will be illustrated by way of examples, which must however not be construed as limiting the scope of invention.

### EXAMPLES

### Catalyst preparation

### Preparation example 1: Unsupported catalyst

1 g (24 mmol) cyanamide (Aldrich) was molten and stirred at 70 °C and subsequently 2 g Ludox HS40 (an aqueous dispersion of 40 wt.-% 12 nm silica particles; manufactured by Aldrich) was added dropwise. The resulting transparent mixture was then heated at a rate of 4.5 K min⁻¹ over 2 h to reach a temperature of 550 °C and then tempered at this temperature for another 4 h. Then, the resulting powder was treated overnight with a. 4M aqueous NH₄HF₂ to remove the silica particle template. The powder was then centrifuged and washed three times with distilled water and twice with ethanol. Finally, the powders were dried at 70 °C under vacuum for several hours. The obtained porous carbonitride had a specific surface area of 190 m²/g⁻¹ and an average pore diameter of 12 nm.

### Preparation example 2: Supported catalyst

As supporting material, silica mesoporous microspheres were used (LiChrosphere Si 100 5µm, Merck, charge F 311016, Art 16116). The silica spheres were dispersed in an aqueous cyanamide solution with varying mass ratios of cyanamide and water and the mixture was sonicated for 10 minutes. The silica spheres were removed by centrifugation (10 minutes; 7000 rpm). The infiltrated spheres were then placed in a closed crucible and heat treated under nitrogen at 550 °C for 4 hours with a heating ramp of 5 °C per minute, to obtain a yellow powder. FT-IR spectroscopy, besides the Si-O vibrations showed the characteristic bands of graphitic C₃N₄. Nitrogen adsorption measurements showed the porosity of the resulting powders and the increase of the surface area by decreasing the cyanamide concentration. The below Table 1 shows the effects of the varying mass ratios of cyanamide and water in the aqueous cyanamide solution on the BET specific surface area of the obtained graphitic C₃N₄. In the Table "CA" means cyanamide.

**Table 1: effect of cyanamide concentration on surface area of graphitic C₃N₄.**

| Mass CA/Mass H₂O (g/g) | Mass ratio CA/H₂O | Specific BET surface area (m²/g) |
|---|---|---|
| 4/2 | 2/1 | 125 |
| 2/2 | 1/1 | 155 |
| 1/2 | 1/2 | 211 |
| 0.65/2 | 1/3.1 | 258 |

### Preparation Example 3: Supported catalyst

The method of preparation Example 2 was repeated except that in place of the silica mesoporous microspheres corresponding amine functionalized silica spheres were used. These amine functionalized silica spheres were prepared as follows.

160µL of 3-(aminopropyl)-triethoxysilane (APTES) were dissolved in 100 ml dry toluene. 1g of the Si100 5µm Merck particles were added. The mixture was stirred at room temperature over night. The product was then collected by suction filtration and washed with 1 1 deionised water and air dried.

The obtained powders showed the same spectroscopic properties as the samples obtained in preparation Example 2. Nitrogen absorption measurements gave similar results.

The average pore diameter was determined by recording TEM (transmission electron microscopy) images on a Zeiss EM91 microscope. The specific surface area of the catalyst was measured according to the BET method using nitrogen adsorption. No trace of iron or other transition metals could be detected by Energy Dispersive X-ray analysis (EDX). FT-IR spectra were recorded on a BioRad FTS600 spectrometer using an attenuated total reflexion (ATR) set-up.

### Catalytic tests

The reactants of the catalytic experiments were generally used as purchased and without further purification.

### 1. Alkyne and nitrile cyclizations

All alkyne cyclisations were carried out in a glass tube sealed with a Teflon cap, with magnetic stirring. All nitrile cyclisations were conducted without stirring in a 40 ml high pressure reactor. The mesoporous carbonitride obtained in preparation example 1 was charged, with a subsequent addition of the starting alkyne or nitrile, and then, optionally, the solvent. The details of the reactions are shown in Table 2, below.

**Table 2: Results of alkyne and nitrile cyclizations**

| **Educt (amount, g)** | **Carbonitride (amount, g)** | **Solvent (volume), ml)** | **Temp. (°C)** | **Reaction time (h)** | **Conversion Rate¹ (%)** | **TON²** | **TOF³ (h-1)** | **Products (selectivity)** |
|---|---|---|---|---|---|---|---|---|
| acetonitrile (10) | 50 | / | 180 | 80 | 20 | 181 | 2.3 | 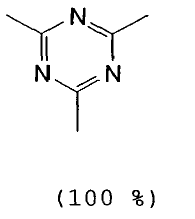 |
| acetonitrile (10) | 50 | / | 150 | 48 | 1 | 2.3 | 0.05 | 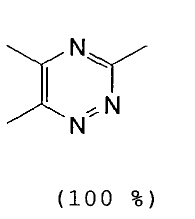 |
| propionitrile (1.1) | 100 | hexane (5) | 180 | 48 | 15 | 5 | 0.1 | 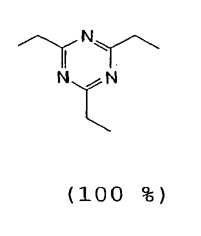 |
| Pyrazine carbonitrile (1.0) | 100 | hexane (5) | 180 | 48 | 100 | 17.6 | 0.37 | 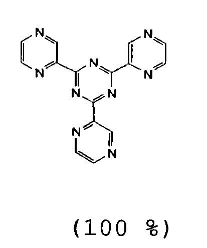 |
| 3-Chloropropionitrile (0.9) | 50 | Hexane (5) | 180 | 48 | 10 | 2.3 | 0.05 | 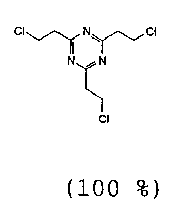 |
| Phenylacetylene (3.0) | 50 | / | 140 | 24 | 8 | 2.6 | 0.12 | 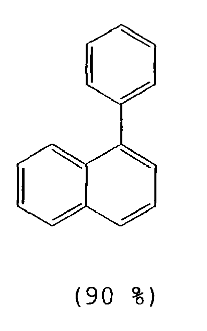 |
| | | | | | | | | 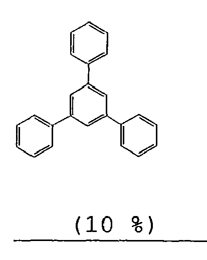 |
| Phenylacetylene (3.0) | 50 | / | 180 | 24 | 75 | 81 | 3.4 | 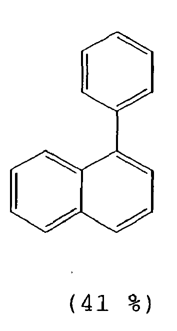 |
| | | | | | | | | 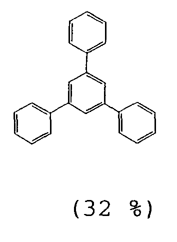 |
| Diphenylacetylene (0.5) | 50 | Xylene (5) | 150 | 60 | 100 | 11 | 0.18 | 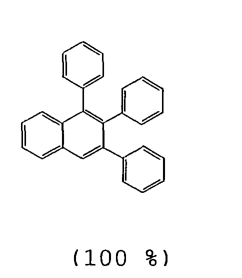 |
| Dimethylacetylenedicarboxylate (0.5) | 50 | Xylene (5) | 120 | 48 | 10 | 0.26 | 0.01 | 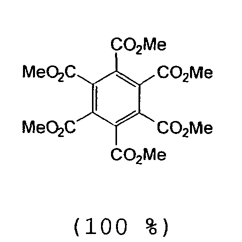 |
| Propargyl alcohol (0.2) | 50 | Xylene (5) | 120 | 24 | 70 | Not determined | Not determined | 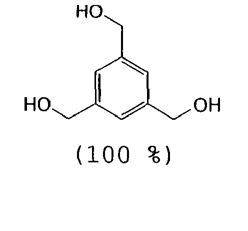 |

| | | | | | | | | |
|---|---|---|---|---|---|---|---|---|
| 1 Conversion rates refer to the conversion rates after terminating the reaction. Further catalyst addition and longer reaction times increase the conversion rate in all cases. 2 Turnover number (number of reacted molecules per catalytic unit) was calculated on the basis of the C₆N₈ units. The theoretical amount of C₆N₈ units was determined on the basis of the initial mass of catalyst and its stoichiometry as determined by elemental analysis. 3 Turnover frequencies (number of converted molecules per hour and catalytic unit) | | | | | | | | |

### 2. Synthesis of dimethylcarbonate

50 mg of porous C₃N₄ obtained in preparation example 1 were washed tree times with a 2.5 molar KOH water solution and dried in vacuo overnight. The powder was then placed in a 100 ml stainless steel autoclave. The autoclave was Teflon^{®}-jacketed to avoid any contamination by transition metals, and 20 ml of methanol were added. The closed autoclave was pressurized to 5 bars with CO. After 40 h reaction at 150°C under magnetic stirring, 30 mol% of CO had been converted to dimethyl carbonate (450 mg).

### 3. Reaction of benzene with carbon dioxide

Using porous C₃N₄ obtained in preparation example 1 as a catalyst, benzene was reacted with carbon dioxide from various CO₂ sources, such as NaHCO₃ or gaseous CO₂.

The reactions were carried out in a 100 ml Teflon^{®}-jacketed stainless steel autoclave, an inside thermoregulation, a 60 bars manometer and a magnetic stirrer (Berghof, BR-100). When the used CO₂ source was NaHCO₃ the given amount of salt was directly placed in the autoclave, the porous C₃N₄ (100 mg), the solvent (either heptane or the wanted aromatic compound; 10 ml) was then added, the reactor closed and heated up to 150 °C. When the CO₂ source was gaseous CO₂, porous C₃N₄ (100 mg) was placed in the reactor together with the solvent and the required amount of triethylamine, and the reactor was closed. The autoclave was then flushed three times with CO₂ and pressurized with CO₂ (to 10 bars) after what it was heated up to 150 °C.

The reaction mixture was neutralized with 1 molar HCl and directly injected in the GC-MS (Agilent Technoligies, GC 6890N, MS 5975). The products were isolated by extraction with toluene. After evaporation of the solvent in vacuo, ¹H- and ¹³C-NMR spectra were recorded in CDCl₃ (Bruker DMX 400) to ascertain the product distribution.

The results of the above reactions are shown in Table 3.

**Table 3: Reaction of benzene and CO₂ from various sources**

| | **CO₂ source** | **Solvent** | **Co-reactant** | **Time (h)** | **Conversion Rate (%)¹** | **Product²** |
|---|---|---|---|---|---|---|
| 1 | / | Benzene | Triethylamine (200 mg) | 20 | 0 | / |
| 2 | NaHCO₃ | Benzene | / | 20 | 46³ | Phenol |
| | (200 mg) | | | | | (100%) |
| 3 | NaHCO₃ | Benzene | / | 20 | 100³ | Phenol |
| | (100 mg) | | | | | (100%) |
| 4 | NaHCO₃ | Heptane | Benzene | 20 | 100⁴ | Phenol |
| | (400 mg) | | (100 mg) | | | (100%) |
| 5 | CO₂ (10 bars) | Benzene | Triethylamine | 12 | 20⁵ | Phenol |
| | | | | | | (65%) |
| | | | (200 mg) | | | Biphenyl |
| | | | | | | (35%) |
| 6^{*} | CO₂ (10 bars) | Benzene | Triethylamine | 24 | 0 | / |
| | | | (200 mg) | | | |

| | | | | | | |
|---|---|---|---|---|---|---|
| ¹ Conversion rates were determined by GC-MS with internal standard as the ratio between the formed products and the initial amount of limiting reactant. ² The percentage inside the brackets is the amount of the specified product among the various reaction products as determined by GC-MS with internal standard. ³ The conversion rate was calculated with respect to the initial amount of NaHCO_{3.} ⁴ The conversion rate was calculated with respect to the amount of benzene. ⁵ The conversion rate was calculated with respect to the amount of base. * Reference test without catalyst. | | | | | | |

### 4. Reaction of benzene with carbonyl compounds

Generally, 50 mg porous C₃N₄ obtained in preparation Example 1 were used, and the reaction conditions were 150 °C and 24 h.

200 mg acetic acid in 5 ml benzene yielded 100 % phenol at a conversion rate of 0.4 %. The reaction of 100 mg acetaldehyde and 200 mg benzene in 5 ml hexane as a solvent yielded 100 % phenol at a conversion rate of 21 %. 200 mg hexanal in 5 ml benzene were reacted to give phenol apart from 1-phenylhexanol and p-dihydroxyhexylbenzene. The conversion rate in this reaction was 90 %.

## Claims

1. A use of a carbonitride as a catalyst.

2. The use according to Claim 1, wherein the catalyst contains less than 0.1 % by weight of transition metal.

3. The use according to Claim 1 or 2, wherein the carbonitride is metal-free.

4. The use according to any one of Claims 1 to 3, wherein the catalyst is an unsupported catalyst.

5. The use according to any one of Claims 1 to 4, wherein the carbonitride is a graphitic carbonitride represented by the formula C₆N₈₊ₓH_{y}, wherein x is 0 to 1 and y is 0 to 3.

6. The use according to Claim 5, wherein the graphitic carbonitride has a specific BET surface area of 50 to 500 m²/g.

7. The use according to any one of Claims 1 to 4, wherein the carbonitride is represented by the formula C₁NₐH_{b}, wherein a is 0.5 to 2 and b is 0 to 1.

8. The use according to any one of Claims 1 to 7, wherein more than 99 % by weight of the catalyst consist of the carbonitride.

9. A method for the cyclization of at least one organic compound each having at least one triple bond, which method comprises contacting the at least one organic compound with a catalyst as defined in any one of Claims 1 to 8.

10. The method of Claim 9 which is for the cyclization of two or three organic compounds each having one triple bond.

11. The method of Claim 9 which is for the cyclization of three organic compounds each having one triple bond.

12. The method of any one of Claims 9 to 11, wherein the triple bond is a carbon-carbon or carbon-nitrogen triple bond.

13. The method of Claim 12, wherein the product of the method is an aromatic compound.

14. The method of Claim 13 wherein the aromatic compound obtained as the product of the method is represented by any one of the following formulae (I), (II) or (III): 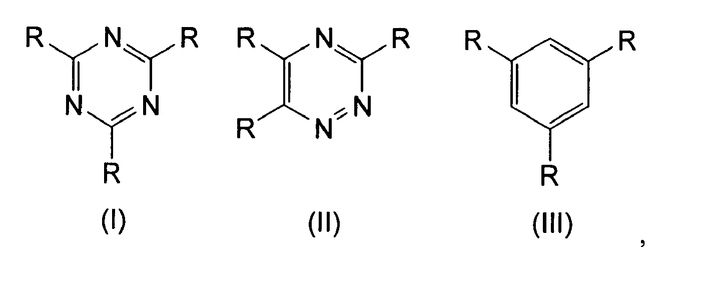 wherein R is independently selected from optionally substituted C₁₋₆ alkyl, C₃₋₁₀ aryl, C₃₋₁₀ heteroaryl and CO₂A with A representing C₁₋₆ alkyl.

15. A method for the preparation of a phenolic compound which comprises reacting the corresponding aromatic compound with carbon dioxide, a carboxylic acid, an aldehyde, an amide or a ketone in the presence of a catalyst as defined in any one of Claims 1 to 8.

16. The method of Claim 15, wherein the aromatic compound is reacted with carbon dioxide.

17. The method of Claim 16, wherein the carbon dioxide is added in the form of a hydrogen carbonate.

18. The method of Claim 16 or 17, wherein a base is present in the reaction mixture.

19. The method of Claim 18, wherein the base has a pKₐ value of at least 11.

20. The method of Claim 15, wherein the aromatic compound is reacted with a carboxylic acid, which is a C₁₋₁₀ carboxylic acid.

21. The method of Claim 15, wherein the aromatic compound is reacted with an aldehyde which is a C₁₋₁₀ aldehyde.

22. The method of Claim 15, wherein the aromatic compound is reacted with a ketone which is a C₃₋₁₀ ketone.

23. The method of any one of Claims 15 to 22, wherein the aromatic compound is an unsubstituted aromatic compound.

24. The method of any one of Claims 15 to 23, wherein the phenolic compound is phenol, and the aromatic compound is benzene.

25. A method for the preparation of a compound represented by formula (IV): 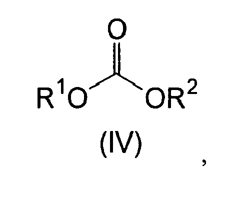 wherein R¹ and R² are the same or different and can be selected from the group consisting of alkyl, aryl and heteroaryl, and can mutually combine to form a ring structure containing the -OCOO-moiety, which comprises reacting carbon monoxide with at least one corresponding alcohol in the presence of a catalyst as defined in any one of Claims 1 to 8.

26. The method of Claim 25, wherein the compound of formula (IV) is dimethyl carbonate and the alcohol is methanol.

27. A supported catalyst comprising a supporting material, and a carbonitride provided thereon.

28. The supported catalyst of Claim 27 which has a specific BET surface area of 200 to 300 m²/g.

29. A method of preparing a supported catalyst according to Claims 27 or 28, which comprises the following steps:
(i) dispersing the supporting material in a solution of the molecular precursor compounds of the carbonitride to infiltrate the molecular precursor compounds in the supporting material;
(ii) recovering the infiltrated supporting material from the solution; and
(iii) heat treating the recovered infiltrated supporting material to convert the molecular precursor compounds to the carbonitride.

30. The method of Claim 29 wherein the supporting material is silica and the solution of the molecular precursor compounds of the carbonitride is an aqueous solution.

31. The process according to Claim 30, wherein the mass ratio of the molecular precursor compounds to water in the aqueous solution is 1:2 to 1:4.

32. The process according to any one of Claims 29 to 31, wherein the molecular precursor compound is cyanamide.
